Europäisches Patentamt

European Patent Office

Office européen des brevets

(11) Publication number : **0 474 347 A1**

(12)

# EUROPEAN PATENT APPLICATION

(21) Application number : **91306489.5**

(22) Date of filing : **17.07.91**

(51) Int. Cl.⁵ : **C12P 23/00,** C12N 1/06,
C12N 15/01, C12N 1/16,
// (C12P23/00, C12R1:645)

(30) Priority : **20.07.90 EP 90307989**

(43) Date of publication of application :
**11.03.92 Bulletin 92/11**

(84) Designated Contracting States :
**AT BE CH DE DK ES FR GB GR IT LI NL SE**

(71) Applicant : **QUEST INTERNATIONAL B.V.**
**Huizerstraatweg 28**
**NL-1411 GP Naarden (NL)**

(72) Inventor : **Chapman, John William**
**4 Ladywood, Clackmannan**
**Clackmannanshire, Scotland (GB)**
Inventor : **Hakkaart, Marcellinus Jacobus J.**
**Surinamelaan 18**
**NL-1213 VN Hilversum (NL)**
Inventor : **Marsden, William James Newton**
**25 Inverallan Drive, Bridge of Allan**
**Stirlingshire, Scotland FK9 4JR (GB)**
Inventor : **Maume, Kate**
**Ostsingel 8**
**NL-3441 GC Woerden (NL)**

(74) Representative : **Hartong, Richard Leroy et al**
**Unilever N.V. Patent Division P.O. Box 137**
**NL-3130 AC Vlaardingen (NL)**

(54) Astaxanthin-generating yeast cells.

(57) Yeast cells capable of generating at least 1100 μg astaxanthin per gram and useful as a fish feed are disclosed. Mutagenisis of Phaffia rhodozyma using EMS and/or NTG is a preferred route to obtain these cells. Rupturing of the cells to release the pigment makes use of an alpha 1,3 glucanase containing enzyme. Methods of drying, concentrating and storing the cells are described.

EP 0 474 347 A1

The present invention relates to yeast cells capable of generating high levels of astaxanthin, methods for producing such cells, to methods for extracting astaxanthin therefrom and to methods for drying, preserving and concentrating such cells.

The red skin and flesh colour of naturally occurring salmon and trout is due to carotenoids, primarily astaxanthin, which is usually present as pigment in these fishes. In nature, marine organisms in the diet provide salmon with these carotenoid pigments.

Due to a lack of dietary astaxanthin, fish raised on fish-farms or in hatcheries are generally pale and lack the skin and flesh colours characteristic of fish grown in their natural environment. Since the colour of a food is frequently an indicator of its quality, there is a strong consumer preference for fish having natural coloration, even though nutritionally the pale farm produced fish may be identical to those grown in their natural environment, although some believe that astaxanthin has other functions. Thus there is evidence that astaxanthin or its precursor contributes to the distinctive flavour of baked salmon.

It is known to feed synthetic astaxanthin and analogues thereof to improve their colour. However, there is an increasing consumer preference for the use of natural rather than synthetic colouring agents in food. The use of synthetic astaxanthin may also be subject to legislative restrictions.

Certain yeasts of the Phaffia genus are known to produce low levels of astaxanthin. Mutants of naturally occurring yeasts have been described in the literature, allegedly capable of generating higher levels of astaxanthin. So far as we are aware, the best yeast strain deposited in a culture collection is CBS 215-88 described in Example 6 of International Patent Application No. WO88/08025 (Danisco) capable, according to the test method described therein, of generating 880 μg of astaxanthin per gram of yeast dry matter. In order to make the feeding of astaxanthin generating yeasts to fish economic and to avoid feeding large quantities of yeast for cost reasons, it would be necessary to raise the level of astaxanthin in the feed and, therefore, yeast strains capable of generating still higher levels of astaxanthin would be of value. Reports of mutant yeast strains having the capability of generating still higher levels of astaxanthin have not been supported by adequate disclosure or by deposition in a culture collection.

We have now discovered yeast strains capable of generating more than 1000 μg/g astaxanthin and methods for producing such yeast strains. Such strains have been found to exhibit particularly high growth rates.

Thus, according to the invention there is provided a yeast cell capable of generating at least 1000 μg/g of astaxanthin based on the yeast dry matter, when subjected to the test conditions specified herein.

The test by which the level of astaxanthin capable of being generated is as follows.

The yeast cells under test are firstly grown under conditions comprising an oxygen transfer rate of at least 30 mmoles/l/hour in YM broth (Difco 0711-01) at 21°C for 60 hours. 500 ml baffled shake flasks are used, containing 50 ml of the medium. The flasks are agitated on an orbital shaker with a 35mm orbit at 160 rpm. The cell walls are ruptured using NovoZym 234 (ex Novo Nordisk A/S, Denmark). Astaxanthin is taken up into a chloroform solvent, sufficient solvent being used to extract all the pigment, a typical amount being a concentration of 1 gm of product per 10 mls of solvent, and the solution is subjected to HPLC analysis. As a standard "Carophyll Pink 5%" (ex Hoffman La Roche) is used, which is presumed to contain 5% trans-astaxanthin. The major peak of the sample at 489 nm is compared with the major peak of the standard. Where a standard containing astaxanthin at a level other than 5% is used, an appropriate correction should be made.

We prefer yeast strains which, under these test conditions, generate at least 1100 μg/g, most preferably at least 1400 μg/g. At these levels the use of such yeast strains in fish feed becomes commercially attractive.

We have also discovered a process for producing yeast strains which may lead to mutations capable of generating at least 1000 μg/g astaxanthin under our test conditions.

Thus according to a second aspect of the invention there is provided a method producing yeast strains according to the first aspect of the invention comprising subjecting Phaffia rhodozyma to a one or more stage mutagenesis, using as mutation agent an agent capable of causing heritable damage to DNA. Such a mutation agent may be ultra violet light, ethyl methane sulphonate (EMS) or N-methyl-N-nitro-N-nitrosoguanidine (NTG).

The mutagenesis may be carried out in one or more stages. After treatment with these agents at a dose sufficient to kill at least 60% of the cells, preferably at a dose sufficient to kill 90% of the cells, the yeast is allowed to recover and express mutations by growth, for example by growth in a rich medium such as YEPD (= yeast extract/bactopeptone/dextrose at 1%/2%/2%). The success of these techniques is surprising, particularly in the production of stable mutations, ie mutations having a low reversion frequency.

A possible specific method of carrying out this process is to use EMS mutagenesis in a first stage and to subject the product thereof to a second stage mutagenesis with NTG. The first stage may be replaced by culturing the Phaffia rhodozyma in the presence of an antibiotic known to inhibit the electron transport chain, such as antimycin A, oligomycin, valinomycin or rotenone but we have not found that this produces a stable material.

The EMS mutagenesis may be carried out in a buffered medium at a pH of 6.5 to 7.5, preferably using a phosphate buffer. EMS is added at a concentration of from 2% to 8%. The treatment time may be from 5 to 30 minutes. After standing and washing the mutated cells may be plated out onto YM agar plates containing antibiotics and other inhibiting mutagenic agents.

The NTG mutagenesis may be carried out in a buffered medium at a pH of 5 to 7.5, preferably using sodium citrate solution as a buffer. NTG is added to give a concentration of from 0.002 to 0.006%. A convenient method of adding the NTG is in the form of a sodium citrate solution. Following mixing and incubation for 20 to 30 minutes, the supernatant may be discarded and the cells washed, preferably in a potassium phosphate buffer at pH 7.0. The yeast strains obtained may be grown in YM broth and are then plated onto an enriched medium, such as YEPD agar.

Following mutation, useful mutant strains can be identified by a screening technique. The yeasts are then plated on YEPD agar to allow the development of single colonies. After 5 days growth at 20 - 25°C, the colour of the colonies can be assessed and any which appear to be more highly coloured than a control wild-type yeast culture may be subcultured to single colonies on YEPD agar.

The stability of the mutant yeast may be assessed by repeated subculture in liquid media. Single colonies may be used to inoculate 7 ml tubes of YEPD broth and these cultures may then be incubated, with shaking, at 20 - 25°C for three days. A 0.1 ml sample may then be used to inoculate a subsequent liquid culture which may also be grown for 3 days. At intervals throughout this process, samples are taken and plated on YEPD agar to count the proportion of darker coloured yeast colonies within the population. Any yeast which, after 3 such subcultures, fails to give greater than 90% more highly coloured colonies is considered to be too unstable to be commercially useful.

The yeast strains according to the invention may be stored in the usual manner. For example the strains may be maintained on slants of YM agar, comprising 10 g/l dextrose, 3 g/l yeast extract, 3 g/l malt extract, 5 g/l bacteriological peptone and 20 g/l agar. Storage in this manner is possible at +4°C for up to 1 month. The strains may also be freeze-dried.

Longer term storage is possible in liquid nitrogen at -196°C in YM culture broth containing 10 vol% dimethyl sulphoxide.

Fermentation of the yeast strains may be carried out in the usual manner, with an initial culture stage, a seed culture stage, a seed fermentation stage and a product fermentation stage. The fermentation stages may involve the need to include an antifoam such as dimethyl polysiloxane. Correct screening of the yeast strains means that this scale-up of production is more likely to be successful.

In order to release the astaxanthin it will be preferable to rupture the cells. While this may be carried out using physical methods such as a conventional bead mill, such as a KDL-Special Dynomill, autolysis and chemical methods are also possible. We prefer to use an enzymatic method.

According to a third aspect of the invention we provide a method of extracting astaxanthin from the yeast cells by rupturing the cell walls by treatment with an enzyme selected from alpha 1,3 glucanase, beta 1,3 glucanase, laminarinase, xylanase, chitinase, protease and mixtures thereof. The combination of these enzymes is available as for example Novozym 234, ex Novo Nordisk A/S. To carry out the cell rupture process, a buffered solution of the selected enzyme or enzymes may be prepared, with a pH of from 4.0 to 6.0. Depending on the activity of the enzyme, we have found that a concentration equivalent from 2.5 to 10 mg of Novozym 234 per 100 mg dry weight of cells is suitable.

The cells are incubated in the enzyme/buffer solution at a temperature of from 30°C to 50°C, for from 1 to 3 hours. Occasional mixing is recommended.

The ruptured cells may be dried by conventional techniques, such as spray drying, fluid bed drying, freeze drying or roller bed drying, with an optional physical concentration step before drying. Drying aids can be added. Filtration of the liquid would leave some dead yeast materials in solution, which could be used for other purposes, such as as animal feed or as a food flavour.

From the ruptured cells, the required pigment may be extracted, for example with the aid of a solvent. Many solvents are suitable including hydrocarbons, esters, alcohols, ketones and vegetable oils, preferably food acceptable solvents such as hexane, cyclohexane, butyl acetate, ethyl acetate, iso-propyl alcohol, ethanol, acetone, methyl ethyl ketone, palm oil or coconut oil. Chloroform and dichloro methane may also be used. Extraction is carried out by the addition of the solvent to the ruptured cells, preferably dried, followed by thorough mixing. The solvent layer can be removed following separation by centrifugation. The extraction may be repeated until no further colour remains in the cell mass and the solvent is clear.

The concentration of astaxanthin may be increased by removing some yeast cell wall material by dissolution, followed by physical separation to yield yeast cell wall material having a relatively high level of astaxanthin associated therewith.

It will be advisable to stabilise pigment in the yeast cells and cell debris during and after rupturing the cell wall, which is sensitive to oxidation and heat. The addition of an anti-oxidant is effective, directly into the bead mill or into the enzyme solution, according to whichever method of cell rupture is used. Oxygen scavengers, free radical scavengers, chelating agents and sequestrants may be used. The anti-oxidant is preferably oil-soluble and is added in the form of an aqueous emulsion. The preferred anti-oxidant is delta tocopherol. Other examples include BHA (butylhydroxyanisol), BHT (butyl hydroxytoluene), vitamin E or ascorbic acid.

In order to prevent the pigment from breaking down on introduction into the aqueous phase, eg. as a fish feed, it is advisable to incorporate the pigment in a suitable encapsulation or delivery system. Such measures may also serve to improve the uptake and passage of the pigment in the alimentary system of the fish, to prevent early breakdown while allowing uptake into the required tissues. To this end, the stabilised pigment maybe encapsulated or aggregated with gelatinized starch. Such encapsulates preferably have a size of less than 1 mm, preferably less than 0.01mm. Ideally, they are generally spherical with a large surface area and insoluble but digestible (eg by the use of pH sensitive encapsulation material).

In addition to use in a fish feed, the astaxanthin derived from the yeast strains according to the invention may be used as a colouring agent in animal feeds especially poultry feed, or in food, cosmetic and health care products for human consumption, such as edible oils, butter, margarine, shortening, mayonnaise, pates, soups, snack products, desserts, ice cream, bakery products, beverages, tanning aids, lipsticks and the like.

The invention will now be described in the following Examples.

### Examples 1 and 2

Samples of a known yeast, Phaffia rhodozyma, ATCC 24202, were subjected to two stage mutation as follows:

Cells (0.3 mg/ml) were placed in a test tube containing a phosphate buffer (pH 7.0). Ethyl methane sulphonate was added to give a 4% concentration mixed with the cells. After 15 minutes standing the cells were washed in a buffer solution approximately 6 times. The cells were plated out onto YM agar plates.

The spun down cells from the first stage mutation were washed in a sodium citrate solution (0.1M pH 5.0). NTG in sodium citrate was added to the solution to give a concentration of 40 μg/ml. Following mixing and incubation for 25 minutes, the supernatant was decontaminated using hydrochloric acid and discarded and the cells were washed with potassium phosphate buffer pH 7.0. The cells were resuspended and grown overnight in YM broth, before plating on to YEPD.

The best yeast strain determined by visual observation obtained in this manner was coded QST 11032.

To produce a second yeast strain, code QST 10984, the first stage EMS treatment was replaced with the following.

The cells were plated out onto YM agar plates containing 50 μM antimycin A (Sigma Chemicals A 8674) and incubated at 21°C for 6 weeks. The resulting viable cultures were then subjected to NTG mutagenesis as described above.

The resultant highly coloured colonies were grown up under the following conditions.

The cells were grown at 21°C in YM broth, 50 mls volume being used in a 500 ml flask which was agitated for 60 hours at 160 rpm on an orbital shaker with a 35mm orbit. The medium was unbuffered. These conditions are sufficient to obtain an oxygen transfer rate of at least 30 mmoles/l/hour.

To rupture the grown cells and release the astaxanthin, a solution containing alpha 1,3 glucanase was prepared at 8% concentration in a buffer at pH 4.2. The cells are added at the concentration of 100 mg dry weight to 10 mg enzyme. An equivalent amount of the enzyme/buffer solution was then added and incubation took place at 42°C for 1.5 hours, mixing occasionally. The ruptured cells are removed, dried and then subjected to the following test procedure.

From the ruptured cells the pigment is extracted using a chloroform solvent (5% w/v). The chloroform is added to the ruptured cells followed by thorough mixing. The mixture is centrifuged and the solvent layer removed. Delta tocopherol is added as an antioxidant (200 ppm). Extraction is continued until no further colour is remaining in the cell mass, and the extracting solvent is also clear.

HPLC is carried out on the extracted pigment solution using a 25 cm long 4.6 mm diameter column filled with Nucleosil 100-5 (silica) column material coated by pumping through the column 30 ml of 1% orthophosphoric acid in methanol at 0.5 ml/min. After injection of 10 μl of sample, the column was eluted with a dichloromethane/2-propanol/n-hexane (10:2:88 w/v%) mixture at 1.8 ml/min for 15 minutes. A UV detector is used, set at 489 nm. As a standard, Carophyll Pink 5% ex Hoffmann La Roche is used.

The two mutant yeast strains, QST 10984 and QST 11032, have been deposited under the terms of the Budapest Treaty in the culture collection NCYC, Norwich accession numbers NCYC 2341 (deposited 11 October 1989) and NCYC 2342 (deposited 24 November 1989) respectively.

The results are set out below, the natural Phaffia rhodozyma ATCC 24202 being included for comparison.

| Sample | Astaxanthin μg/g yeast dry matter |
|--------|-----------------------------------|
| ATCC 24202 | 325 |
| QST 10984 | 1100 |
| QST 11032 | 1400 |

The strains were maintained on slants of YM agar (Difco 0712-01-8) having the following composition:

10 g/l dextrose
3 g/l yeast extract
3 g/l malt extract
5 g/l bacteriological peptone
20 g/l agar

Following storage at about +4°C the strains were recultivated on fresh slants as described. The strains were grown on in a number of stages as follows:

i) In YM broth (Difco 0711-01) for 60 hours at 21°C.

ii) 3% inoculum from stage (i) was added to a baffled shake flask, 50 ml liquid medium in a 500 ml volume flask which was incubated on an orbital shaker, 35 mm orbit at 160 rpm for 60 hours at 21°C. The pH was not controlled.

iii) 1% inoculum from stage (ii) was added to YM broth at 21°C for 48 hours. Aeration took place in this stage at a rate of 0.1 vvm to give an oxygen transfer of more than 30 mol/m3 hr. The pH was controlled at 5.0 during this stage, by the addition of 4M KOH or 10% tartaric acid as appropriate. Dimethyl polysiloxane antifoam (0.5%) was also added.

iv) 3% inoculum from stage (iii) was added to 60 g/l molasses (4:1 beet:blackstrap or 3:2 as an alternative) at 21°C, the pH being controlled at 5.0. Aeration at a rate of 0.6 vvm to give an oxygen transfer of more than 30 mol/m3. hr. Dimethypolysiloxane antifoam (0.5%) was also added.

A 120 litre fermenter is half filled with the substrate and a start medium comprising 6.5 g/l yeast extract, 0.7 g/l ammonium sulphate, 0.23 g/l diammonium hydrogen phosphate and 3 mg/l hydrated zinc sulphate. After 60 hours, when a yeast dry matter concentration of 1 g/l was achieved, further substrate and necessary nutrient requirements at the same concentrations were pumped in over a 40 hours period to supplement those nutrients already metabolised. Aeration is continued for a further 24 hour period.

The cells are harvested as follows:

Cells were collected by centrifugation washed thoroughly in order to remove any growth medium or inhibitory material. The cells were then dried on roller bed driers and stored.

## Example 3

### Fish Feeding Trials

The trial was designed to evaluate the yeast by incorporating it into the diets of rainbow trout. Such a trial would give an indication of the suitability of the product for salmonids in gross nutritional, total growth and histopathological terms. The trial would also provide samples for the determination of the pigment uptake and deposition by the fish.

Phaffia rhodozyma mutations described in Examples 1 & 2 were used and compared against a synthetic source of astaxanthin.

Yeasts were subjected to pre-treatment prior to drying, including mechanical homogenisation, enzyme degradation and osmotic shock.

Antioxidants such as delta-tocopherol, ascorbyl palmitate, citric acid, ascorbic acid and stabilisers such as sorbitan monostearate, sorbitan monopalmitate, carboxymethyl cellulose and gelatin were added in order to protect the pigment during the drying as well as during subsequent processing into fish feed. The yeast was treated and dried in a manner which would enhance the uptake and incorporation of the carotenoids into the fish flesh.

Fish feeding diets were compounded with each ingredient within the range of inclusion level currently in use by commercial feed manufacturers. Feed components which included brown fish meal, soya bean meal, wheat meal, vitamins, mineral binders, fish oils and chromic oxide (as a digestibility marker) were thoroughly mixed and pelleted through a mill with a 4 mm diameter die hole size.

The proximate composition of the diet can be described as moisture 6%; protein 50%; lipid 13%; ash 11% and fibre 3.0%.

A control feed containing no added pigment was also generated.

The fish were maintained under normal tank husbandry conditions for rainbow trout and fed on a daily ration calculated from feed tables common in the industry.

No abnormal fish behaviour was observed during the trial. The fish fed readily on the experimental diets. The gain in individual mean wet body weight over the trial did not differ significantly between the different diets. Food conversion ratio, protein efficiency ratio and apparent net protein utilisation showed little variation between the diets. There was no abnormal histopathology. The fish were healthy and of good body condition at the start and finish of the trial.

The inclusion of yeast feedstuffs in the diets had no detectable detrimental effect on growth, nutritional performance, health or carcass composition of rainbow trout.

There was no significant difference between any of the diets (P>0.05) indicating that the diets performed virtually equally.

The apparent pigment digestibility ranged up to 73%, with the yeast based feedstuffs being between 58% to 73%.

Fish flesh and skin were examined for astaxanthin content. For flesh, a 10 g fillet was taken and cut into small pieces and placed in a mortar to which 20 g of anhydrous $Na_2SO_4$ was added. The material was ground and then acetone soluble material was extracted. The extract was filtered to remove solids and evaporated in a rotary vacuum evaporator. The resulting coloured fish oil was dissolved in chloroform and the trans-astaxanthin content assessed by recommended HPLC analysis method.

All flesh samples possessed trans-astaxanthin greater than 2 ppm. All treatments produced fish with flesh containing more trans-astaxanthin than the control which received no pigments in the diet ration.

## Claims

1. A yeast cell capable of generating at least 1000 μg/g of astaxanthin based on the yeast dry matter, when subjected to the test conditions specified herein.

2. Yeast strains according to Claim 1 deposited under accession Nos. NCYC 2341 and NCYC 2342 (QST 10984 and 11032) and mutants or derivatives thereof having retained astaxanthin generating capability.

3. A yeast cell according to Claim 1, capable of generating at least 1400 μg/g of astaxanthin based on the yeast dry matter.

4. Dead yeast cell wall material having associated therewith at least 1000 μg/g of astaxanthin based on the yeast dry matter.

5. A method of producing a yeast cell according to Claim 1, which comprises subjecting Phaffia rhodozyma to a one or more stage mutagenesis, using a mutation agent capable of causing heritable damage to DNA.

6. A method according to Claim 5, wherein Phaffia rhodozyma is subjected to a dose of mutation agent sufficient to kill 60% of the cells, the remaining cells being allowed to recover and express mutations by growth.

7. A method of extracting astaxanthin from yeast cells according to Claim 1, including the step of rupturing the cell walls by treatment with an enzyme from selected alpha 1,3 glucanase, beta 1,3 glucanase, laminarinase, xylanase, chitinase, protease and mixtures thereof.

8. Use of yeast cells containing at least 1000 μg/g of astaxanthin, based on the yeast dry matter, as a fish feed, in animal feed or in food products for human consumption.

9. Use of astaxanthin extracted from yeast cells according to Claim 1, as fish feed, in animal feed or in food products for human consumption.

EP 0 474 347 A1

**European Patent Office**

**EUROPEAN SEARCH REPORT**

Application Number

EP 91 30 6489

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | WO-A-8 808 025 (DANISCO BIOTEKNOLOGI A/S)<br>* the whole document * | 1, 8, 9 | C 12 P 23/00<br>C 12 N 1/06<br>C 12 N 15/01<br>C 12 N 1/16 //<br>(C 12 P 23/00<br>C 12 R 1:645) |
| X,A | WO-A-9 001 552 (IGENE BIOTECHNOLOGY,INC.)<br>* the whole document * | 1, 3-5,<br>7-9,6 | |
| A | Derwent Publications Ltd.,London, GB, DATABASE WPIL, accession no.83-10788k,Week 8305 & JP-A-57206342 (SANRAKU OCEAN) 17.12.82 | 7 | |
| A | APPLIED AND ENVIRONMENTAL MICROBIOLOGY vol. 35, no. 6, June 78, WASHINGTON US pages 1155 - 1159; E.A.JOHNSON ET AL.: "SIMPLE METHOD FOR THE ISOLATION OF ASTAXANTHIN FROM THE BASIDIOMYCETOUS YEAST PHAFFIA RHODOZYMA "<br>* abstract * | 1, 2, 7 | |
| A | THE JOURNAL OF GENERAL MICROBIOLOGY vol. 115, no. 1, November 79, COLCHESTER GB pages 173 - 183; E.A.JOHNSON ET AL.: "ASTAXANTHIN FORMATION BY THE YEAST PHAFFIA RHODOZYMA"<br>* abstract * | 1, 2, 7 | TECHNICAL FIELDS SEARCHED (Int. Cl.5)<br><br>C 12 P<br>C 12 N<br>C 12 R |
| A | AQUACULTURE vol. 20, 1980, AMSTERDAM pages 124 - 129; Johnson E.A. et al.: "Phaffia rhodozyma as an astaxanthin source in salmonid diets"<br>* page 124, line 17 - page 129, line 19 * | 1, 5, 8,9 | |

The present search report has been drawn up for all claims

| Place of search | Date of completion of the search | Examiner |
|---|---|---|
| BERLIN | 18-10-1991 | GURDJIAN D P M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

7

**European Patent Office**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF APPLIED BACTERIOLOGY vol. 59, 1985, Oxford GB pages 243 - 255; R.N.Okagbue et al.: "Influence of mixed culture conditions on yeast-wall hydrolytic activity of Bacillus circulans WL-12 and on extractability of astaxan -thin from the yeast Phaffia rhodozyma" * the whole document * | 7, 8 | |
| | | | TECHNICAL FIELDS SEARCHED (Int. Cl.5) |

The present search report has been drawn up for all claims

| Place of search | Date of completion of the search | Examiner |
|---|---|---|
| BERLIN | 18-10-1991 | GURDJIAN D P M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................
& : member of the same patent family, corresponding document

EPO FORM 1503 03.82 (P04101)